# EUROPÄISCHE PATENTSCHRIFT

(11) **EP 4 014 907 B1**
(45) Veröffentlichungstag und Bekanntmachung des Hinweises auf die Patenterteilung: **17.04.2024**
(21) Anmeldenummer: 21214533.8
(22) Anmeldetag: 14.12.2021
(51) Int. Cl.: A61B 18/12, A61B 18/00

(54) **ELEKTROCHIRURGIEGENERATOR**
ELECTROSURGICAL GENERATOR
GÉNÉRATEUR D'ÉLECTROCHIRURGIE

(30) Priorität: 17.12.2020 DE 102020134062
(43) Veröffentlichungstag der Anmeldung: 22.06.2022
(73) Patentinhaber: Olympus Winter & Ibe GmbH, 22045 Hamburg (DE)
(72) Erfinder: DIJKSTRA, Jelle, 10781 Berlin (DE); SCHIDDEL, Stefan, 14532 Stahnsdorf (DE)
(74) Vertreter: Eisenführ Speiser

(56) Entgegenhaltungen:
- CN-A- 112 071 595
- DE-C- 614 728
- DE-T5-112009 001 250
- US-A- 4 739 759

## Beschreibung

Die Erfindung betrifft einen Elektrochirurgiegeneratorzum Bereitstellen eines hochfrequenten Wechselstromes/ einer hochfrequenten Wechselspannung, mit wenigstens zwei Ausgängen, an die ein elektrochirurgisches Instrument angeschlossen ist oder angeschlossen werden kann, wenigstens einer hochfrequenten Spannungsquelle und wenigstens zwei Ausgangstransformatoren, die primärseitig mit der hochfrequenten Spannungsquelle und sekundärseitig mit den Ausgängen für ein elektrochirurgisches Instrument verbunden sind.

Mittels Elektrochirurgie kann biologisches Gewebe - also Körpergewebe - geschnitten, koaguliert (verödet) und/oder vaporisiert werden. Zu diesem Zweck wird in der Regel eine hochfrequente Wechselspannung/ ein hochfrequenter Wechselstrom über ein elektrochirurgisches Instrument in das Körpergewebe geleitet, wobei das elektrochirurgische Instrument mit den Ausgängen eines Elektrochirurgiegenerators verbunden ist.

Bekannte Elektrochirurgiegeneratoren sind so ausgebildet, dass sie verschiedene Betriebsmodi anbieten, so dass mit einem Elektrochirurgiegenerator sowohl geschnitten, koaguliert als auch vaporisiert werden kann. Je nach Anwendung (Schneiden, Koagulieren oder Vaporisieren) sind bestimmte hochfrequente Wechselspannungen/ Wechselströme (im Folgenden vereinfachend Ausgangsspannung genannt) erforderlich. Um eine bestimmte Ausgangsspannung bereitstellen zu können, weisen bekannte Elektrochirurgiegeneratoren eine hochfrequente Spannungsquelle auf, die aus einer Gleichspannung eine sinusförmige Hochfrequenzspannung (200 kHz - 5 MHz) erzeugt. Die hochfrequente Spannungsquelle umfasst in der Regel eine mit einem Wechselrichter verbundene Spannungsquelle. Um an den Ausgängen des Elektrochirurgiegenerators die jeweils geforderte Ausgangsspannung, die je nach Anwendung von mehreren Volt bis zu mehreren Kilovolt variieren kann, bereitzustellen, ist weiterhin ein Ausgangstransformator zwischen der hochfrequenten Spannungsquelle und den Ausgängen für ein elektrochirurgisches Instrument angeordnet, über den eine Skalierung der Ausgangspannung erfolgt und der weiterhin eine galvanische Trennung zum Patientenkreis gewährleistet.

Um den geforderten Ausgangsspannungsbereich abzudecken, sind unterschiedliche Transformatorverhältnisse zwischen der hochfrequenten Spannungsquelle und den Ausgängen für ein elektrochirurgisches Instrument erforderlich. Es sind verschiedene Konzepte bekannt, um mehrere Transformatorverhältnisse innerhalb eines einzelnen Elektrochirurgiegenerators bereitstellen zu können.

So ist zum Beispiel die Verwendung eines Ausgangstransformators bekannt, der mehrere Sekundärwicklungen aufweist, die in Reihe geschaltet sind. Weiterhin auch bekannt ist die Verwendung eines Ausgangstransformators, der mehrere in Reihe geschaltete Primärwicklungen aufweist. Diese beiden Konzepte weisen den Nachteil auf, dass auch an den Leerlaufwicklungen, die nicht verwendet werden, Spannungen auftreten, weil sie magnetisch mit der Primärwicklung gekoppelt sind. Diese Spannungen verursachen eine hohe Belastung des Dielektrikums zwischen den Wicklungen und können die Patientenleckströme des Generators erhöhen. Außerdem wird der Transformator aufgrund der mehreren Abgriffe sehr komplex.

Ein weiteres Konzept sieht vor, dass in einer elektrochirurgischen Einheit für jedes gewünschte Übersetzungsverhältnis ein Ausgangstransformator vorgesehen ist. Dieses Konzept weist den Nachteil auf, dass für die verschiedenen Ausgangstransformatoren sehr viel Platz im Generator benötigt wird, obwohl für die Verwendung eines Betriebsmodus jeweils nur ein Transformator erforderlich ist. Entsprechend werden die Ausgangstransformatoren nicht effizient eingesetzt.

Ein Beispiel eines elektrochirurgischen Generators mit schaltbaren Ausgangstransformatoren wird in US 4739579 offenbart.

Der Erfindung liegt die Aufgabe zugrunde, das Anwendungsspektrum eines Elektrochirurgiegenerators zu erweitern.

Erfindungsgemäß ist vorgesehen, dass sowohl die Primärwicklungen der Ausgangstransformatoren des Elektrochirurgiegenerators über Schalter miteinander verbunden werden können als auch die Sekundärwicklungen der Ausgangstransformatoren des Elektrochirurgiegenerators über Schalter miteinander verbunden werden können.

Bei der vorliegenden Erfindung ist erkannt worden, dass eine sinnvolle Kombination der aus dem Stand der Technik bekannten Konzepte durchaus vorteilhaft sein kann, denn die Verwendung von mehreren Ausgangstransformatoren in Verbindung mit Schaltmöglichkeiten, die bei Bedarf vorgegebene Primärwicklungen der Ausgangstransformatoren miteinander verbindet und/ oder Sekundärwicklungen der Ausgangstransformatoren miteinander verbindet, ermöglicht in einfacher Weise das Herstellen unterschiedlicher Transformatorverhältnisse zwischen der hochfrequenten Spannungsquelle und den Ausgängen für ein elektrochirurgisches Instrument ohne die aus dem Stand der Technik bekannten Nachteile. Zudem ergibt die erfindungsgemäße Kombination aus wenigstens zwei Ausgangstransformatoren, deren Primär- und Sekundärwicklungen über Schalter miteinander gekoppelt werden können, eine Transformatorkombination, die sich nach außen wie ein einzelner Transformator verhält, ohne dass ungenutzte Leerlaufspannungen entstehen.

Gemäß einer bevorzugten Ausgestaltung kann die Varianz der unterschiedlichen Transformatorverhältnisse noch erhöht werden, wenn eine Kopplung der Primärwicklungen bzw. der Sekundärwicklungen der Ausgangstransformatoren über die Schalter so gestaltet ist, dass die Primärwicklungen und die Sekundärwicklungen jeweils wahlweise parallel zueinander und/oder in Reihe geschaltet oder einzeln betrieben werden können.

Gemäß einer weiteren Ausgestaltung der Erfindung kann wenigstens ein Ausgangstransformator auf der Primär- und/ oder Sekundärseite mindestens eine Anzapfung aufweisen. Diese Ausgestaltung ermöglicht eine weitere Varianz der Übersetzungsverhältnisse. So kann zu Beispiel mit Hilfe von Mittelabgriffen das Betreiben von Instrumenten mit mehr als zwei Elektroden erreicht werden.

Das Öffnen und Schließen der Schalter erfolgt über eine Steuereinheit. Die Steuereinheit enthält oder erhält von einer weiteren Steuereinheit die Informationen, wann eine Betätigung der Schalter erfolgen soll. Vorzugsweise erfolgt das Betätigen der Schalter, wenn ein Betriebsmodus ausgewählt wurde und das Instrument in Betrieb genommen wird. Denkbar wäre aber auch, das vorab festgelegt und abgespeichert worden ist, in welcher Reihenfolge die Schalter geöffnet bzw. geschlossen werden müssen, um bestimmte Übersetzungsverhältnisse zu erhalten.

In einer bevorzugten Ausgestaltung weist der erfindungsgemäße Elektrochirurgiegenerator drei Ausgangstransformatoren mit jeweils unterschiedlichem Übersetzungsverhältnis auf, wobei der mittlere Trafo zum Erhalt der richtigen Anordnung der Polaritätspunkte der einzelnen Ausgangstransformatoren entweder primär oder sekundär "gedreht" werden muss. Die drei Primärwicklungen der Einzeltransformatoren sind jeweils in Serie miteinander geschaltet. Auch die drei Sekundärwicklungen der Einzeltransformatoren sind jeweils in Serie miteinander geschaltet. Sowohl primärseitig als auch sekundärseitig sind jeweils wenigstens zwei Schalter vorgesehen, die so mit den Primärwicklungen bzw. den Sekundärwicklungen verbunden sind, dass
mit einem ersten Schalter die erste und die zweite Primärwicklung überbrückt werden, wenn der erste Schalter geschlossen ist,
mit einem zweiten Schalter die zweite und die dritte Primärwicklung überbrückt werden, wenn der zweite Schalter geschlossen ist,
mit einem dritten Schalter die erste und die zweite Sekundärwicklung überbrückt werden, wenn der dritte Schalter geschlossen ist, und
mit einem vierten Schalter die zweite und die dritte Sekundärwicklung überbrückt werden, wenn der erste Schalter geschlossen ist.

Hierzu ist der erste Schalter der ersten und der zweiten Primärwicklung parallel geschaltet. Der zweite Schalter ist der zweiten und der der dritten Primärwicklung parallel geschaltet. Der dritte Schalter ist der ersten und der zweiten Sekundärwicklung parallel geschaltet. Der vierte Schalter ist der zweiten und der dritten Sekundärwicklung parallel geschaltet.

Vorzugsweise weist der Elektrochirurgiegenerator eine Steuereinheit zum Ansteuern der vier Schalter auf. Die Steuereinheit ist ausgebildet, durch entsprechendes Öffnen oder Schließen der Schalter vier verschiedene Betriebsmodi bereitzustellen.

In einem ersten Betriebsmodus sind die beiden primärseitigen Schalter geöffnet und sämtliche Primärwicklungen Serie geschaltet. Gleichzeitig sind die beiden sekundärseitigen Schalter geschlossen und die drei Sekundärwicklungen sind parallel zueinander geschaltet.

In einem zweiten Betriebsmodus ist der erste primärseitige Schalter, der der ersten Primärwicklung und der zweiten Primärwicklung parallel geschaltet ist, geöffnet, während der zweite primärseitige Schalter, der der zweiten und dritten Primärwicklung parallel geschaltet ist, geschlossen ist. Gleichzeitig ist der erste sekundärseitige Schalter, der der ersten und zweiten Sekundärwicklung parallelgeschaltet ist, geöffnet und der zweite sekundärseitige Schalter, der der zweiten und dritten Sekundärwicklung parallelgeschaltet ist, ist geschlossen.

In einem dritten Betriebsmodus ist der erste primärseitige Schalter, der der ersten Primärwicklung und der zweiten Primärwicklung parallel geschaltet ist, geschlossen und der zweite primärseitige Schalter, der der zweiten und dritten Primärwicklung parallel geschaltet ist, ist geöffnet. Gleichzeitig ist der erste sekundärseitige Schalter, der der ersten und zweiten Sekundärwicklung parallel geschaltet ist, geschlossen und der zweite sekundärseitige Schalter der der zweiten und dritten Sekundärwicklung parallel geschaltet ist, ist geöffnet.

In einem vierten Betriebsmodus sind die beiden primärseitigen Schalter geschlossen und die drei Primärwicklungen sind zueinander parallelgeschaltet. Gleichzeitig sind die beiden sekundärseitigen Schalter geöffnet und die drei Sekundärwicklungen sind in Reihe geschaltet.

Gemäß einer weiteren vorteilhaften Ausgestaltung der Erfindung sind die Übersetzungsverhältnisse der beiden äußeren Ausgangstransformatoren unterschiedlich ausgebildet. Diese Ausgestaltung bietet den Vorteil, dass diese beiden Ausgangstransformatoren auch einzeln geschaltet werden können. Das Übersetzungsverhältnis des mittleren Ausgangstransformators sollte dann in der Größenordnung des kleineren Übersetzungsverhältnisses der beiden äußeren Ausgangstransformatoren liegen.

Um die Aktivierung von zwei elektrochirurgischen Instrumenten zu ermöglichen, kann der erfindungsgemäße Elektrochirurgiegenerator wenigstens zwei hochfrequente Spannungsquellen aufweisen, wobei die Spannungsquellen über Schalter mit den Primärwicklungen der Ausgangstransformatoren verbunden werden können.

Vorzugsweise sind die Schalter als Relais ausgeführt.

Der Elektrochirurgiegenerator ist vorzugsweise dazu konfiguriert, an seinen Ausgängen eine Ausgangswechselspannung mit einer Frequenz zwischen 0,2 MHz und 3 MHz bereitzustellen.

Der Elektrochirurgiegenerator ist vorzugsweise dazu konfiguriert, an seinen Ausgängen eine Ausgangswechselspannung zwischen 20 V und 5 kV bereitzustellen.

Der Elektrochirurgiegenerator ist vorzugsweise dazu konfiguriert, an seinen Ausgängen eine Ausgangsleistung von bis zu 500 W bereitzustellen.

Der Elektrochirurgiegenerator ist vorzugsweise dazu konfiguriert, Betriebsmodi sowohl zum Schneiden als auch zum Koagulieren von Körpergewebe bereitzustellen.

Ein derartig konfigurierter Elektrochirurgiegenerator kann in Verbindung mit wenigstens zwei Ausgangstransformatoren, die nach außen wie ein einziger Ausgangstransformator wirken universell und wirtschaftlich betrieben werden.

Die Erfindung soll nun anhand eines Ausführungsbeispiels mit Bezug auf die Figuren näher erläutert werden. Von den Figuren zeigt
- Fig. 1:: eine schematische Darstellung einiger Komponenten eines Elektrochirurgiegenerators zur Versorgung eines elektrochirurgischen Instruments mit einer hochfrequenten Wechselspannung;
- Fig. 2:: eine schematische Darstellung einer ersten Ausführungsform eines von drei in Reihe geschalteten Einzeltransformatoren gebildeten Ausgangstransformators des Elektrochirurgiegenerators aus Figur 1;
- Fig. 3:: eine schematische Darstellung einer zweiten Ausführungsform eines von drei in Reihe geschalteten Einzeltransformatoren gebildeten Ausgangstransformators in einem ersten Betriebsmodus;
- Fig. 4:: eine schematische Darstellung einer zweiten Ausführungsform eines von drei in Reihe geschalteten Einzeltransformatoren gebildeten Ausgangstransformators in einem zweiten Betriebsmodus;
- Fig. 5:: eine schematische Darstellung einer zweiten Ausführungsform eines von drei in Reihe geschalteten Einzeltransformatoren gebildeten Ausgangstransformators in einem dritten Betriebsmodus;
- Fig. 6:: eine schematische Darstellung einer zweiten Ausführungsform eines von drei in Reihe geschalteten Einzeltransformatoren gebildeten Ausgangstransformators in einem vierten Betriebsmodus; und
- Fig. 7:: eine schematische Darstellung einer zweiten Ausführungsform eines von drei in Reihe geschalteten Einzeltransformatoren gebildeten Ausgangstransformators in einem fünften Betriebsmodus.

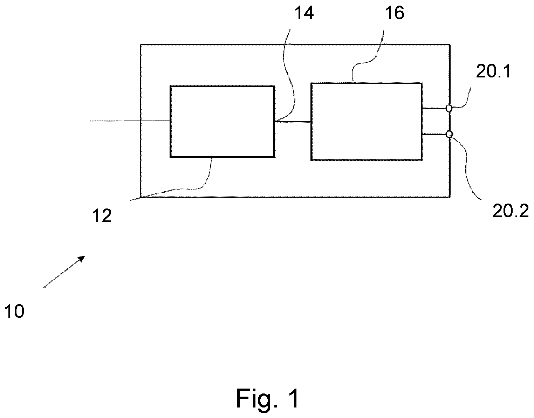

Figur 1 zeigt ein Beispiel für einen erfindungsgemäßen Elektrochirurgiegenerator 10. Wie Figur 1 zu entnehmen ist, weist der Elektrochirurgiegenerator 10 eine hochfrequente Spannungsquelle 12 auf, die beispielsweise an das übliche öffentliche Stromversorgungsnetz angeschlossen werden kann und an ihrem Ausgang 14 eine Ausgangsgleichspannung bereitstellt. Die Ausgangsgleichspannung wird einem Hochfrequenzteil 16 des Elektrochirurgiegenerators 10 zugeführt. Der Hochfrequenzteil 16 des Elektrochirurgiegenerators 10 wirkt als Wechselrichter und erzeugt eine hochfrequente Ausgangswechselspannung, die an Ausgänge 20.1 und 20.2 des Elektrochirurgiegenerators 10 abgegeben wird. An die Ausgänge 20.1 und 20.2 des Elektrochirurgiegenerators 10 kann ein elektrochirurgisches Instrument angeschlossen werden.

Der Hochfrequenzteil 16 des Elektrochirurgiegenerators 10 ist beispielsweise dafür ausgelegt, einen hochfrequenten Wechselstrom und eine hochfrequente Wechselspannung mit einer Frequenz zwischen 200 kHz und 5 MHz abzugeben. Die gewünschte Amplitude der Ausgangswechselspannung kann zwischen einigen Volt und einigen Kilovolt variieren und hängt von der jeweiligen Anwendung ab.

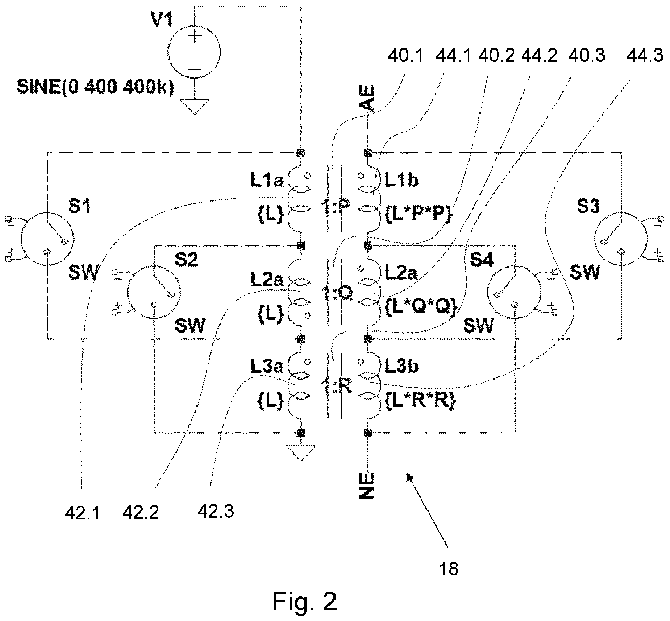

Um einen großen Bereich möglicher Ausgangsspannungen zu erzielen, umfasst der Elektrochirurgiegenerator in dem in Figur 2 dargestellten Ausführungsbeispiel drei Einzeltransformatoren 40.1, 40.2 und 40.3. Jeder Einzeltransformator 40.1, 40.2 und 40.3 weist jeweils eine Primärwicklung 42.1, 42.2 und 42.3 sowie eine Sekundärwicklung 44.1, 44.2 und 44.3 auf. Sowohl die drei Primärwicklungen 42.1, 42.2 und 42.3 als auch die drei Sekundärwicklungen 44.1, 44.2 und 44.3 sind jeweils zueinander in Reihe geschaltet, wobei wobei der mittlere Trafo 40.2 zum Erhalt der richtigen Anordnung der Polaritätspunkte der einzelnen Ausgangstransformatoren auf der Primärseite gedreht wurde.

Jeder der Einzeltransformatoren 40.1, 40.2 und 40.3 des in Figur 2 dargestellten Ausführungsbeispiels hat ein anderes Übersetzungsverhältnis. Das jeweilige Übersetzungsverhältnis ergibt sich aus dem Verhältnis der Anzahl der Windungen der jeweiligen Primärwicklung zur Anzahl der Windungen der entsprechenden Sekundärwicklung. Dabei ist das Verhältnis der Eingangsspannung zur Ausgangsspannung des jeweiligen Einzeltransformators 40.1, 40.2 und 40.3 umgekehrt proportional zum Verhältnis der Anzahl der Windungen der Primärwicklung zur Anzahl der Windungen der Sekundärwicklung. Das Übersetzungsverhältnis - dies ist das Verhältnis von Eingangsspannung zur Ausgangsspannung des jeweiligen Einzeltransformators - ist für die Einzeltransformatoren 40.1, 40.2 und 40.3 in Figur 2 jeweils als 1:P, 1:Q beziehungsweise 1:R angegeben.

Neben den drei Einzeltransformatoren 40.1, 40.2 und 40.3 sind sowohl primärseitig - also auf Seiten der Primärwicklungen 42.1, 42.2 und 42.3 - als auch sekundärseitig - also auf Seiten der Sekundärwicklungen 44.1, 44.2 und 44.3 - jeweils zwei Schalter vorgesehen, die in ihrem geschlossenen Zustand jeweils zwei derdrei Wicklungen überbrücken können. Konkret ist ein Schalter S1 zu den Primärwicklungen 42.1 und 42.2 parallelgeschaltet und kann diese beiden Primärwicklungen überbrücken. Ein Schalter S2 ist den Primärwicklungen 42.2 und 42.3 parallelgeschaltet und kann diese beiden Primärwicklungen überbrücken.

Ähnlich sind sekundärseitig ebenfalls zwei Schalter S3 und S4 vorgesehen, von denen der Schalter S3 den Sekundärwicklungen 44.1 und 44.2 parallelgeschaltet ist und der Schalter S4 den Sekundärwicklungen 44.2 und 44.3 parallelgeschaltet ist.

Mit Hilfe der primärseitigen Schalter S1 und S2 und der sekundärseitigen Schalter S3 und S4 kann zwischen verschiedenen Übersetzungsverhältnissen umgeschaltet werden.

Wenn beispielsweise in einem ersten Betriebsmodus die beiden primärseitigen Schalter S1 und S2 geöffnet sind, sind sämtliche Primärwicklungen 42.1, 42.2 und 42.3 in Serie geschaltet. Wenn gleichzeitig die beiden sekundärseitigen Schalter S3 und S4 geschlossen sind, sind die drei Sekundärwicklungen 44.1, 44.2 und 44.3 parallel zueinander geschaltet. Mit geöffneten primärseitigen Schaltern S1 und S2 und geschlossenen sekundärseitigen Schaltern S3 und S4 ergibt sich ein Übersetzungsverhältnis mit 1:(1/P + 1/Q + 1/R). Dies ist das niedrigste mögliche Übersetzungsverhältnis, was auch den geringsten Magnetisierungsstrom erfordert.

Wenn in einem zweiten Betriebsmodus der erste primärseitige Schalter S1, der den beiden Primärwicklungen 42.1 und 42.2 parallel geschaltet ist, geöffnet ist, während der zweite primärseitige Schalter S2, der den Primärwicklungen 42.2 und 42.3 parallel geschaltet ist, geschlossen ist, ist nur die Primärwicklung 44.1 des ersten Trasformators 40.1 aktiv, weil die beiden anderen Primärwicklungen 42.2 und 42.3 von dem zweiten primärseitigen Schalter S2 überbrückt sind. Wenn gleichzeitig der erste sekundärseitige Schalter S3, der den Sekundärwicklungen 44.1 und 44.2 parallel geschaltet ist, geöffnet ist und der zweite sekundärseitige Schalter S4, der den Sekundärwicklungen 44.2 und 44.3 parallel geschaltet ist, geschlossen ist, ist auch sekundärseitig nur die erste der Sekundärwicklungen 44.1 aktiv, weil die beiden übrigen Sekundärwicklungen 44.2 und 44.3 durch den Schalter S4 überbrückt sind. In diesem Fall entspricht das Übersetzungsverhältnis des Ausgangstransformators 18 dem Übersetzungsverhältnis des ersten Einzeltransformators 40.1, und ist entsprechend 1:P.

Wenn in einem dritten Betriebsmodus der erste primärseitige Schalter S1 geschlossen und der zweite primärseitige Schalter S2 geöffnet ist, ist primärseitig nur die dritte Primärwicklung 44.3 aktiv, weil der erste primärseitige Schalter S1 die beiden übrigen Primärwicklungen 42.1 und 42.2 überbrückt. Wenn auf der Sekundärseite gleichzeitig der erste sekundärseitige Schalter S3 geschlossen und der zweite sekundärseitige Schalter S4 geöffnet ist, ist auch auf der Sekundärseite nur die dritte Sekundärwicklung 44.3 aktiv, weil der zweite sekundärseitige Schalter S3 die beiden übrigen sekundärseitigen Wicklungen 44.1 und 44.2 überbrückt. Der Ausgangstransformator 18 hat dann ein Übersetzungsverhältnis von 1:R, also das Übersetzungsverhältnis des dritten Einzeltransformators 40.3.

Wenn in einem vierten Betriebsmodus die beiden primärseitigen Schalter S1 und S2 geschlossen sind, sind die drei Primärwicklungen 42.1, 42.2 und 42.3 zueinander parallel geschaltet. Wenn gleichzeitig die beiden sekundärseitigen Schalter S3 und S4 geöffnet sind, sind die drei Sekundärwicklungen 44.1, 44.2 und 44.3 in Reihe geschaltet. In diesem Fall hat der Ausgangstransformator 18 ein Übersetzungsverhältnis von 1:(P + Q + R). Dies ist das höchste Übersetzungsverhältnis, das der Ausgangstransformator 18 haben kann und führt zu dem höchsten Magnetisierungsstrom.

Die Schalter S1, S2, S3 und S4 können beispielsweise Relais aber gegebenenfalls auch Feldeffekttransistoren sein.

Zum Ansteuern der Schalter S1, S3, S3 und S4 ist eine Steuereinheit 46 vorgesehen. Diese ist so konfiguriert, dass sie ein Öffnen und Schließen der entsprechenden Schalter zum Bereitstellen der hier beschriebenen Betriebsmodi bewirkt.

Um in der Praxis beispielsweise gewünschte Übersetzungsverhältnisse von 1:2,6, 1:7,4 und 1:13,4 erzielen zu können, könnten die Werte für P, Q und R, die die Übersetzungsverhältnisse der Einzeltransformatoren 40.1, 40.2 und 40.3 bestimmen, wie folgt gewählt werden: P = 2,6, R = 7,4 und Q = 3,4. Mit diesen Werten kann der Ausgangstransformator 18 auch noch ein viertes Übersetzungsverhältnis von 1 zu 0,8 erzielen.

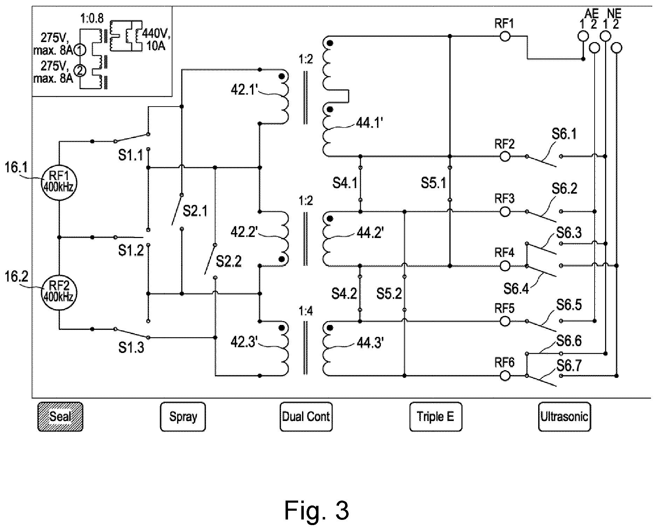

Das in Figur 2 beispielhaft dargestellte Ausführungsbeispiel eines Elektrochirurgiegenerators kann durch geeignete Modifikation so gestaltet sein, dass er auch Betriebsmodi bereitstellt, die den gleichzeitigen Einsatz zweier elektrochirurgischer Instrumente erlauben. Ein entsprechendes Ausführungsbeispiel ist in den Figuren 3 bis 7 dargestellt. Die Figuren 3 bis 7 zeigen jeweils den gleichen Ausgangstransformator in verschiedenen Konfigurationen für verschiedene Betriebsmodi, die durch entsprechende Schalterstellungen von Schaltern des Ausgangstransformators realisiert werden. Ein weiterer Unterschied des in den Figuren 3 bis 7 dargestellten Ausgangstransformators gegenüber dem in Figur 2 dargestellten Ausgangstransformator besteht darin, dass nicht nur ein einziges Paar von Ausgängen 20.1 und 20.2 zum Anschließen einer Arbeitselektrode AE und einer Neutralelektrode NE bereitgestellt sind, sondern jeweils zwei Ausgänge für zwei verschiedene Arbeitselektroden AE1 und AE2 sowie zwei verschiedene Neutralelektroden NE1 und NE2.

Wie das in Figur 2 dargestellte Ausführungsbeispiel weisen auch auch die in den Figuren 3 bis 7 dargestellten Ausführungsbeispiele drei Einzeltransformatoren auf, die jeweils eine Primärwicklung 42.1', 42.2` und 42.3' aufweisen sowie auch jeweils eine Sekundärwicklung 44.1', 44.2` und 44.3'. Auch bei dem in den Figuren 3 bis 7 dargestellten Ausführungsbeispiel eines Ausgangstransformators ist der mittlere Einzeltransformator mit der Primärwicklung 42.2` und gegenüber den beiden übrigen Einzeltransformatoren invertiert.

Die Primärwicklungen 42.1', 42.2' und 42.3' sind genau wie die Primärwicklungen des in Figur 2 dargestellten Ausgangstransformators zueinander in Serie geschaltet. Gleiches gilt auch für die Sekundärwicklungen 44.1', 442.' und 44.3`, wobei im Unterschied zu dem in Figur 2 dargestellten Ausführungsbeispiel zwischen den einzelnen Sekundärwicklungen 44.1', 44.2` und 44.3` Schalter S4.1 und S4.2 vorgesehen sind, mit denen die Serienschaltung zwischen den Sekundärwicklungen 44.1', 44.2' und 44.3' aufgehoben werden kann. Konkret kann mittels des Schalters S4.1 die elektrische Verbindung zwischen der ersten Sekundärwicklung 44.1' und der zweiten Sekundärwicklung 44.2` unterbrochen werden, indem der Schalter S4.1 geöffnet wird. Entsprechend kann die elektrische Verbindung und damit die Serienschaltung zwischen der zweiten Sekundärwicklung 44.2` und der dritten Sekundärwicklung 44.3`durch Öffnen des Schalters S4.2 unterbrochen werden.

Ein weiterer Unterschied zu dem in Figur 2 dargestellten Ausführungsbeispiel besteht darin, dass anstelle eines einzelnen Hochfrequenzteils 16 zwei Hochfrequenzteile 16.1 und 16.2 vorgesehen sind, die zueinander in Serie geschaltet sind, wobei sich durch die Verwendung zweier Hochfrequenzteile 16.1 und 16.2 ein Mittenabgriff ergibt. Drei mit den insgesamt drei Anschlüssen der in Serie geschalteten Hochfrequenzteile 16.1 und 16.2 verbundene Schalter S1.1, S1.2 und S1.3 erlauben es, die Hochfrequenzteile 16.1 und 16.2 entweder in Serie geschaltet oder einzeln mit einer oder mehrerer der Primärwicklungen 42.1', 42.2` und 42.3` zu verbinden.

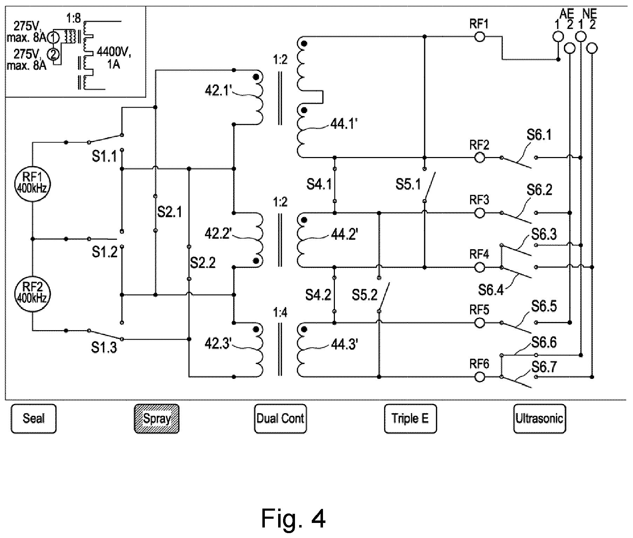

Wenn sich die Schalter S1.1, S1.2 und S1.3 in der in den Figuren 3 und 4 dargestellten Schaltstellung befinden, sind die in Serie geschalteten Hochfrequenzteile 16.1 und 16.2 der Serienschaltung der Primärwicklungen 42.1', 42.2' und 42.3` parallelgeschaltet, sodass die Serienschaltung der Primärwicklungen 42.1', 42.2` und 42.3' von den in Serie geschalteten Hochfrequenzteilen 16.1 und 16.2 in dergleichen Weise mit Spannung versorgt wird, wie die Primärwicklungen 42.1, 42.2 und 42.3 des Ausgangstransformators in Figur 2 von einem einzigen Hochfrequenzteil mit Spannung versorgt werden. Durch die Serienschaltung der Hochfrequenzteile 16.1 und 16.2 stellen diese Hochfrequenzteile 16.1 und 16.2 insgesamt eine Ausgangsspannung bereit, die der Summe der Ausgangsspannungen der beiden einzelnen Hochfrequenzteile 16.1 und 16.2 entspricht.

Wenn ausschließlich die Ausgangsspannung der in Serie geschalteten Hochfrequenzteile 16.1 und 16.2 an der Serienschaltung der Primärwicklungen 42.1', 42.2` und 42.3' anliegen soll, kann der mit dem Mittenabgriff zwischen den beiden Hochfrequenzteilen 16.1 und 16.2 verbundene Schalter S1.2 vollständig geöffnet bleiben, so wie dies in den Figuren 3 und 4 dargestellt ist.

Wenn - wie in Figur 3 abgebildet - die aneinander in Serie geschalteten Hochfrequenzteile 16.1 und 16.2 mittels der Schalter S1.1 und S1.3 mit der Serienschaltung der Primärwicklungen 42.1', 42.2' und 42.3` parallelgeschaltet ist und gleichzeitig die den Schaltern S1 und S2 aus dem Ausführungsbeispiel in Figur 2 entsprechenden Schalter S2.1 und S2.2 geöffnet sind, wirken die Primärwicklungen 42.1', 42.2` und 42.3' in Serie.

Wenn dabei gleichzeitig die Schalter S5.1 und S5.2 zum parallelschalten der Sekundärwicklungen 44.1', 44.2` und 44.3' geschlossen sind, sind die Sekundärwicklungen 44.1', 44.2' und 44.3` zueinander parallelgeschaltet. Das Übersetzungsverhältnis des Ausgangstransformators in dem in Figur 3 dargestellten Betriebsmodus ergibt sich dann aus der Serienschaltung der Primärwicklungen 42.1', 42.2' und 42.3' sowie der Parallelschaltung der Sekundärwicklungen 44.1', 44.2' und 44.3'. Wenn das Übersetzungsverhältnis der ersten beiden Einzeltransformatoren mit ihren Primärwicklungen 42.1' und 42.2' sowie ihren Sekundärwicklungen 44.1' und 44.2`jeweils 1:2 ist und das Übersetzungsverhältnis des dritten Einzeltransformators mit der Primärwicklung 42.3' und der Sekundärwicklung 44.3' 1:4 ist, ergibt sich bei dem in Figur 3 dargestellten Ausführungsbeispiel mit in Serie geschalteten Primärwicklungen 42.1', 42.2', 42.3' und parallelgeschalteten Sekundärwicklungen 44.1', 44.2` und 44.3' insgesamt ein Übersetzungsverhältnis von 1:0,8.

In der oberen linken Ecke der Figur 3 ist ein Ersatzschaltbild dargestellt, das illustriert, wie die Ausgangstransformatoren in dem in Figur 3 dargestellten Ausführungsbeispiel aufgrund der jeweiligen Schalterstellungen im Ergebnis konfiguriert ist.

Die in Figur 3 dargestellte Konfiguration des Ausgangstransformators eignet sich zum Bereitstellen eines Betriebsmodus zum Versiegeln von Gewebe, für das gegebenenfalls ein höherer Ausgangsstrom von beispielsweise maximal 10 A, aber eine nicht ganz so hohe Ausgangsspannung von beispielsweise 440 V benötigt wird.

In dem in Figur 3 dargestellten Ausführungsbeispiel sind die Ausgänge für die zweite Arbeits-elektrode AE2 und die zweite Neutralelektrode NE2 jedoch von den Sekundärwicklungen 44.1', 44.2` und 44.3' getrennt, sodass im Ergebnis nur jeweils ein einziger Ausgang AE1 und NE1 wirksam ist, genau wie dies auch in Figuren 1 und 2 dargestellt ist.

Falls ein zweiter Betriebsmodus eine höhere Ausgangsspannung, dafür aber nicht so einen hohen Ausgangsstrom erfordert, können die in Serie geschalteten Hochfrequenzteile 16.1 und 16.2 mit einer Parallelschaltung der Primärwicklungen 42.1', 42.2` und 42.3' verbunden sein, so wie dies in Figur 4 dargestellt ist. Auch hier bleibt der den Mittenabgriff zwischen den beiden Hochfrequenzteilen 16.1 und 16.2 zugeordnete Schalter S1.2 gänzlich geöffnet. Hinsichtlich der Primärwicklungen 42.1', 42.2` und 42.3' sind die den Schaltern S1 und S2 im Ausführungsbeispiel gemäß Figur 2 entsprechenden Schalter S2.1 und S2.2 zum Parallelschalten der Primärwicklungen 42.1', 42.2' und 42.3' geschlossen. Aufgrund der Tatsache, dass der Schalter S2.1' den Primärwicklungen 42.1' und 42.2` parallelgeschaltet ist und der Schalter S2.2' den Primärwicklungen 42.2' und 42.3` parallelgeschaltet ist, wobei gleichzeitig die mittlere Primärwicklung 42.2' gegenüber den beiden übrigen Primärwicklungen 42.1' und 42.3' invertiert ist, ergibt sich insgesamt die oben links im Ersatzschaltbild dargestellte Parallelschaltung der Primärwicklungen 42.1', 42.2` und 42.3'. Sekundärseitig sind bei dem in Figur 4 dargestellten Ausführungsbeispiel die Sekundärwicklungen 44.1', 44.2' und 44.3' in Serie geschaltet, weil sowohl die Schalter S4.1' und S4.2' geschlossen sind, als auch die Schalter S5.1 und S5.2 geöffnet sind. Mit den Schaltern S5.1 und S5.2 können die Sekundärwicklungen in 44.1', 44.2' und 44.3` parallelgeschaltet werden, wie dies in Figur 3 dargestellt ist. Bei dem Ausführungsbeispiel in Figur 4 ist dies jedoch nicht der Fall.

Auch bei dem Ausführungsbeispiel in Figur 4 wird die Ausgangsspannung nur an Ausgängen für eine erste Arbeitselektrode AE1 und eine erste Neutralelektrode NE1 zur Verfügung gestellt. Die Ausgänge für die zweite Arbeitselektrode AE2 und die zweite Neutralelektrode NE2 sind durch entsprechende Schalter (siehe die Schalter S6.1, S6.2, S6.3, S6.4, S6.5, S6.6 und S6.7) von den Sekundärwicklungen 44.1', 44.2` und 44.3' getrennt.

Bei dem in Figur 4 dargestellten Ausführungsbeispiel ergibt sich bei einem Übersetzungsverhältnis von jeweils 1: 2 für die ersten beiden Einzeltransformatoren mit ihren Primärwicklungen 42.1' und 42.2' sowie ihren Sekundärwicklungen 44.1' und 44.2' und einem Übersetzungsverhältnis von 1: 4 für den dritten Einzeltransformator mit seiner Primärwicklung 42.3` und seiner Sekundärwicklung 44.3' insgesamt ein Übersetzungsverhältnis von 1:8, wenn die Primärwicklungen 42.1', 42.2' und 42.3' einander parallelgeschaltet sind und die Sekundärwicklungen 44.1', 44.2` und 44.3` zueinander in Serie geschaltet sind.

Die in den Figuren 3 und 4 dargestellten Betriebsmodi und Konfigurationen können auch mit der in Figur 2 dargestellten Ausführungsform eines Ausgangstransformators bereitgestellt werden.

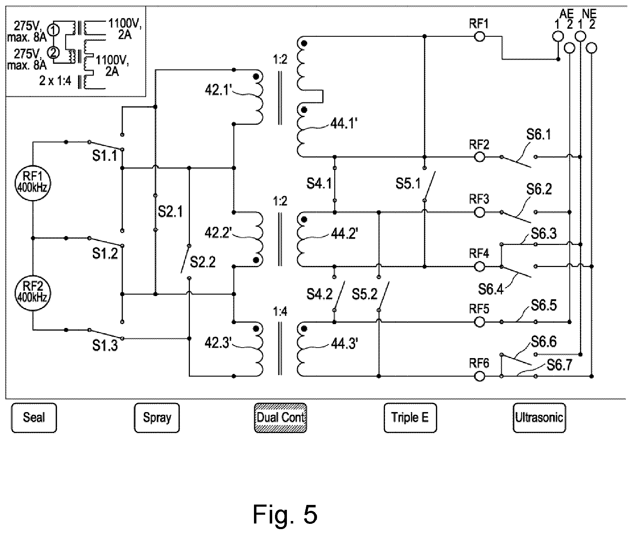

Dadurch, dass bei dem in den Figuren 3 bis 7 dargestellten Ausgangstransformator zwei Hochfrequenzteile 16.1 und 16.2 und die entsprechenden Schalter S1.1, S1.2 und S1.3 vorgesehen sind und außerdem auch am Ausgang des Ausgangstransformators die Schalter S6.1, S6.2, S6.3, S6.4, S6.5, S6.6 und S6.7 zum Verbinden der Anschlüsse der Sekundärwicklungen 44.1', 44.2' und 44.3' mit der ersten und gegebenenfalls auch der zweiten Arbeitselektrode AE1 und AE2 sowie mit der ersten Neutralelektrode NE1 und gegebenenfalls auch der zweiten Neutralelektrode NE2 vorgesehen sind, können mit dem Ausgangstransformator gemäß der Figuren 3 bis 7 auch weitere Betriebsmodi bereitgestellt werden, die mit dem Ausgangstransformator gemäß Figur 2 nicht bereitgestellt werden können. Dies ist in den Figuren 5, 6 und 7 dargestellt, die die Schalterkonfigurationen für die weiteren Betriebsmodi illustrieren.

Die in den Figuren 5, 6 und 7 dargestellten Konfigurationen des Ausgangstransformators erlauben jeweils den Anschluss von zwei elektrochirurgischen Instrumenten für den gleichzeitigen Betrieb. Ein erstes elektrochirurgisches Instrument ist dann an den Anschluss AE1 für die erste Arbeitselektrode und den Anschluss NE1 für die erste Neutralelektrode angeschlossen, während das zweite elektrochirurgische Instrument an den zweiten Anschluss AE2 für die zweite Arbeitselektrode und den zweiten Anschluss NE2 für die zweite Neutralelektrode angeschlossen ist.

Figur 5 zeigt eine Konfiguration des Ausgangstransformators, bei dem ein erstes elektrochirurgisches Instrument über die Anschlüsse AE1 und NE1 von dem ersten Hochfrequenzteil 16.1 über die Primärwicklungen 42.1' und 42.2' sowie die Sekundärwicklungen 44.1' und 44.2' mit Spannung versorgt wird, während ein zweites, an den Anschluss für die zweite Arbeitselektrode AE2 und den Anschluss für die zweite Neutralelektrode NE2 angeschlossenes Instrument von dem zweiten Hochfrequenzteil 16.2 über die dritte Primärwicklung 42.3` und die dritte Sekundärwicklung 44.3' mit Spannung versorgt wird. Der dritte Einzeltransformator wirkt somit tatsächlich als Einzeltransformator, sodass sein Übersetzungsverhältnis von 1:4 unmittelbar zum Tragen kommt. In Bezug auf die übrigen Primärwicklungen 42.1' und 42.2' sowie die übrigen Sekundärwicklungen 44.1' und 44.2` gilt, dass die Primärwicklungen 44.1' und 44.2' durch den geschlossenen Schalter S2.1' parallelgeschaltet sind und bei entsprechender Schalterstellung der Schalter S1.1' und S1.2' von dem ersten Hochfrequenzteil 16.1 mit Spannung versorgt werden. Die zugehörigen Sekundärwicklungen 44.1' und 44.2` sind hingegen in Serie geschaltet, weil der Schalter S4.1 geschlossen und der Schalter S5.1 geöffnet sind. Die Sekundärwicklungen 44.1' und 44.2` sind im Übrigen von der dritten Sekundärwicklung 44.3' aufgrund der geöffneten Schalter S4.2' und S5.2' getrennt. Durch das Parallelschalten der Primärwicklungen 42.1' und 42.2' und die in Serie geschalteten zugehörigen Sekundärwicklungen 44.1' und 44.2' ergibt sich insgesamt ein Übersetzungsverhältnis von ebenfalls 1:4, sodass bei identischen Hochfrequenzteilen 16.1 und 16.2 die beiden an den Anschluss für die erste Arbeitselektrode AE1 und den Anschluss für die erste Neutralelektrode NE1 beziehungsweise den Anschluss für die zweite Arbeitselektrode AE2 und den Anschluss für die zweite Neutralelektrode NE2 angeschlossenen elektrochirurgischen Instrumente mit der gleichen Ausgangsspannung versorgt werden und auch den gleichen maximalen Strom abgeben können.

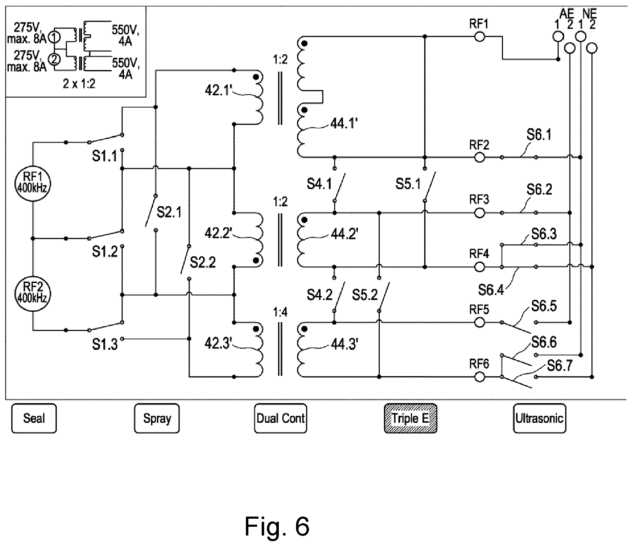

Figur 6 zeigt eine Konfiguration, bei der nur die ersten beiden Einzeltransformatoren mit ihren Primärwicklungen 42.1' und 42.2' sowie die ersten beiden Sekundärwicklungen 44.1' und 44.2` zum Einsatz kommen, während der dritte Einzeltransformator mit seiner dritten Primärwicklung 42.3' und der dritten Sekundärwicklung 44.3` nicht zum Tragen kommt. Bei der in Figur 6 dargestellten Konfiguration wird die erste Primärwicklung 42.1' von dem ersten Hochfrequenzteil 16.1' mit Spannung versorgt, während die zweite Primärwicklung 42.2` von dem zweiten Hochfrequenzteil 16.2' mit Spannung versorgt wird. Die beiden Schalter S2.1 und S2.2 sind entsprechend geöffnet und der mit dem Mittenanschluss zwischen den beiden Hochfrequenzteilen 16.1 und 16.2 verbundene Schalter S1.2 befindet sich einer Schaltstellung, die den Mittenabgriff zwischen den beiden Hochfrequenzteilen 16.1 und 16.2 mit einem Mittenabgriff zwischen den beiden Primärwicklungen 42.1' und 42.2' verbindet. Die beiden Sekundärwicklungen 44.1' und 44.2' wirken jeweils einzeln, weil die Schalter S4.1 und S5.1 geöffnet sind.

Die beiden Schalter S4.2 und S5.2 sind genau wie die Schalter S6.5, S6.6 und S6.7 geöffnet, sodass die dritte Sekundärwicklung 44.3` von den übrigen Komponenten des Transformators und auch den übrigen Anschlüssen des Ausgangstransformators galvanisch getrennt ist.

Bei dem in Figur 6 dargestellten Ausführungsbeispiel wirken somit der erste Einzeltransformator mit seiner Primärwicklung 42.1' und seiner Sekundärwicklung 44.1' und der zweite Einzeltransformator mit seiner Primärwicklung 42.2` und seiner Sekundärwicklung 44.2` unabhängig voneinander. Der erste Einzeltransformator mit seiner Primärwicklung 42.1' und seiner Sekundärwicklung 44.1' versorgt die Anschlüsse für die erste Arbeitselektrode AE1 und für die erste Neutralelektrode NE1, während der zweite Einzeltransformator mit seiner Primärwicklung 42.2` und seiner Sekundärwicklung 44.2' die Anschlüsse für die zweite Arbeitselektrode AE2 und die zweite Neutralelektrode NE2 mit Spannung versorgt. Beide Einzeltransformatoren haben das gleiche Übersetzungsverhältnis von 1:2, sodass die beiden an die erste Arbeitselektrode AE1 und die erste Neutralelektrode NE1 beziehungsweise die zweite Arbeitselektrode AE2 und die zweite Neutralelektrode NE2 angeschlossenen elektrochirurgischen Instrumente wieder mit jeweils der gleichen Spannung versorgt werden.

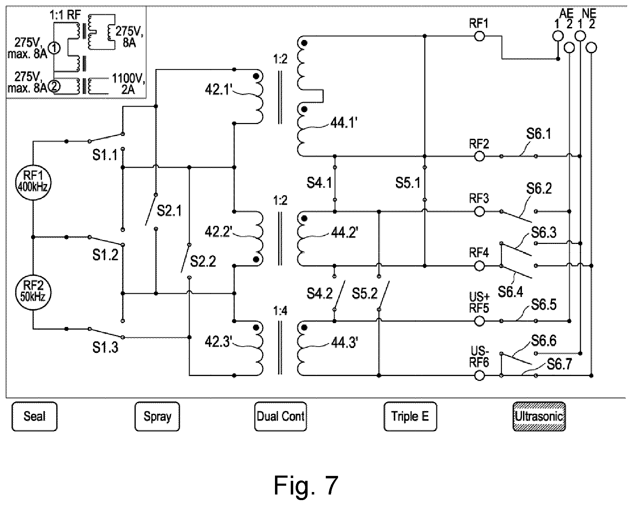

Figur 7 zeigt schließlich eine Konfiguration des in den Figuren 3 bis 7 dargestellten Ausgangstransformators, bei dem zwei an den Anschluss für die erste Arbeitselektrode AE1 und den Anschluss für die erste Neutralelektrode NE1 beziehungsweise den Anschluss für die zweite Arbeitselektrode AE2 und den Anschluss für die zweite Neutralelektrode NE2 angeschlossene elektrochirurgische Instrumente mit unterschiedlicher Spannung versorgt werden. Konkret wird ein erstes, an den Anschluss für die erste Arbeitselektrode AE1 und den Anschluss für die erste Neutralelektrode NE2 angeschlossenes elektrochirurgisches Instrument von dem ersten Hochfrequenzteil 16.1 über eine Serienschaltung der ersten und zweiten Primärwicklung 42.1' und 42.2' sowie eine Parallelschaltung der zugehörigen Sekundärwicklungen 44.1' und 44.2' mit Spannung versorgt. Ein an die Anschlüsse für die zweite Arbeitselektrode AE2 und die zweite Neutralelektrode NE2 angeschlossenes zweites Ultraschallinstrument wird hingegen von dem zweiten Hochfrequenzteil 16.2 mit dem dritten Einzeltransformator mit seiner Primärwicklung 42.3` und seiner Sekundärwicklung 44.3' mit Spannung versorgt. Da in dieser Konfiguration die Schalter S4.2 und S5.2 geöffnet sind, ist die dritte Sekundärwicklung 44.3` von den beiden übrigen Sekundärwicklungen 44.1' und 44.2' getrennt, sodass der dritte Einzeltransformator im Ergebnis unabhängig von den beiden übrigen Einzeltransformatoren wirkt.

In Bezug auf die Anschlüsse für die erste Arbeitselektrode AE1 und die erste Neutralelektrode NE1 gilt, dass deren Versorgung durch das erste Hochfrequenzteil 16.1 und die Serienschaltung der ersten und zweiten Primärwicklungen 42.1' und 42.2' sowie die Parallelschaltung der zugehörigen Sekundärwicklungen 44.1' und 44.2' dadurch bewerkstelligt wird, dass primärseitig der Schalter S2.1 geöffnet ist (auch der Schalter S2.2 ist geöffnet, um die dritte Primärwicklung 42.3' von den übrigen Primärwicklungen 42.1' und 42.2` zu trennen) und sekundärseitig die beiden Schalter S4.1 und S5.1 geschlossen sind.

Durch die in Figur 7 dargestellten Schalterstellungen ergibt sich eine Konfiguration des Ausgangstransformators, die wiederum oben links in Figur 7 in Form eines Ersatzschaltbildes dargestellt ist. Diesem ist zu entnehmen, dass in diesem Fall das an den Anschluss für die erste Arbeitselektrode AE1 und den Anschluss für die erste Neutralelektrode NE1 angeschlossene elektrochirurgische Instrument mit einer Spannung von 275 V versorgt wird und einen maximalen Strom von 8 A abgeben kann. Dies sind genau die Ausgangswerte des ersten Hochfrequenzteils 16.1, da der von den ersten Einzeltransformatoren gebildete Transformator aufgrund der identischen Übersetzungsverhältnisse der beiden ersten Einzeltransformatoren von 1:2 sowie der Serienschaltung der Primärwicklungen 42.1' und 42.2' sowie der Parallelschaltung der zugehörigen Sekundärwicklungen 44.1' und 44.2` insgesamt ein Übersetzungsverhältnis von 1:1 hat.

Aufgrund des Übersetzungsverhältnisses von 1:4 wird das an die Anschlüsse für die zweite Arbeitselektrode AE2 und die zweite Neutralelektrode NE2 angeschlossene elektrochirurgische Instrument mit der vierfachen Ausgangsspannung des zweiten Hochfrequenzteils 16.2 versorgt, im Beispielsfall also mit 1100 V.

Mit einem Elektrochirurgiegenerator mit einem derartigen Ausgangstransformator lassen sich solche Nachteile des Standes der Technik vermeiden, die darauf zurückzuführen sind, dass bei Transformatoren mit verschiedenen Abgriffen (anstelle von Einzeltransformatoren) die nicht genutzten Windungen ebenfalls Spannung führen, weil sie magnetisch mit den übrigen Windungen verknüpft sind. Derartige Spannungen belasten das Dielektrikum zwischen den Windungen sehr und können einen Leckstrom zum Patienten hervorrufen. Außerdem ist ein Transformator mit mehreren Abgriffen komplex.

Anstelle eines umschaltbaren Ausgangstransformators verschiedene individuelle Ausgangstransformatoren vorzusehen, hat den Nachteil, dass dies mehr Raum, mehr Material und mehr Schalter als die erfindungsgemäß vorgeschlagene Lösung erfordert. Auch ist eine Lösung mit verschiedenen Ausgangstransformatoren schon deshalb nicht effizient, weil immer nur einer der Ausgangstransformatoren in Betrieb ist.

### Bezugszeichenliste

- 10: Elektrochirurgiegenerator
- 12: Hochspannungsnetzteil
- 14: Ausgang
- 16: Hochfrequenzteil
- 20.1, 20.2: Ausgänge
- 40.1, 40.2, 40.3: Ausgangstransformatoren
- 42.1, 42.2, 42.3: Primärwicklungen
- 44.1, 44.2, 44.3: Sekundärwicklungen
- 46: Steuereinheit

## Patentansprüche

1. Elektrochirurgiegenerator (10) zum Bereitstellen verschiedener hochfrequenter Wechselspannungen/ hochfrequenter Wechselströme, mit wenigstens zwei Ausgängen (20.1, 20.2), an die ein elektrochirurgisches Instrument angeschlossen werden kann, wenigstens einer hochfrequenten Spannungsquelle (12) und wenigstens zwei Ausgangstransformatoren (40.1, 40.2, 40.3), die jeweils wenigstens eine Primärwicklung (42.1, 42.2, 42.3; 42.1', 42.2', 42.3') und eine Sekundärwicklung (44.1, 44.2, 44.3; 44.1', 44.2', 44.3') aufweisen, wobei die Ausgangstransformatoren (40.1, 40.2, 40.3) primärseitig mit der hochfrequenten Spannungsquelle (12) und sekundärseitig mit den Ausgängen (20.1, 20.2) für ein elektrochirurgisches Instrument verbunden sind,
**dadurch gekennzeichnet, dass** der Elektrochirurgiegenerator (10) Schaltmittel (S1, S2, S2.1, S2.2, S3, S4; S4.1, S4.2, S5.1, S5.2) aufweist, mit denen sowohl die Primärwicklungen (42.1, 42.2, 42.3; 42.1', 42.2', 42.3') der Ausgangstransformatoren (40.1, 40.2, 40.3) wahlweise parallel zueinander und/oder in Reihe geschaltet oder einzeln betrieben werden können als auch die Sekundärwicklungen (44.1, 44.2, 44.3; 44.1', 44.2', 44.3') der Ausgangstransformatoren (40.1, 40.2, 40.3) wahlweise parallel zueinander und/oder in Reihe geschaltet oder einzeln betrieben werden können.

2. Elektrochirurgiegenerator gemäß dem vorhergehenden Anspruch, **dadurch gekennzeichnet, dass** wenigstens ein Ausgangstransformator (40.1, 40.2, 40.3) auf der Primär- und/ oder Sekundärseite mindestens eine Anzapfung aufweist.

3. Elektrochirurgiegenerator gemäß einem der vorhergehenden Ansprüche, **dadurch gekennzeichnet, dass** die Ausgänge der Sekundärwicklungen der Ausgangstransformatoren (40.1, 40.2, 40.3) über Schalter mit den Ausgängen (20.1, 20.2) für ein elektrochirurgisches Instrument verbunden werden können.

4. Elektrochirurgiegenerator gemäß einem der vorhergehenden Ansprüche, **dadurch gekennzeichnet, dass** das Ansteuern der Schaltmittel (S1, S2, S2.1, S2.2, S3, S4, S4.1, S4.2, S5.1, S5.2) über eine Steuereinheit erfolgt.

5. Elektrochirurgiegenerator gemäß einem der Ansprüche 1 oder 2 und 4, **dadurch gekennzeichnet, dass** zum Einstellen von vier verschiedenen Übersetzungsverhältnissen zwischen der hochfrequenten Spannungsquelle und den Ausgängen (20.1, 20.2) für ein elektrochirurgisches Instrument drei Ausgangstransformatoren (40.1, 40.2, 40.3) vorgesehen sind, deren Wicklungen sowohl auf der Primärseite als auch auf der Sekundärseite über jeweils zwei Schalter verbunden werden können.

6. Elektrochirurgiegenerator gemäß dem vorhergehenden Anspruch, **dadurch gekennzeichnet, dass** die Übersetzungsverhältnisse der beiden äußeren Ausgangstransformatoren (40.1, 40.2, 40.3) unterschiedlich ausgebildet sind.

7. Elektrochirurgiegenerator gemäß dem vorhergehenden Anspruch, **dadurch gekennzeichnet, dass** das Übersetzungsverhältnis des mittleren Ausgangstransformators (40.2) in der Größenordnung des kleineren Übersetzungsverhältnisses der äußeren Ausgangstransformatoren (40.1, 40.3) liegt.

8. Elektrochirurgiegenerator gemäß wenigstens einem der vorhergehenden Ansprüche 1 bis 5 **dadurch gekennzeichnet, dass** der Elektrochirurgiegenerator wenigstens zwei hochfrequente Spannungsquellen umfasst.

9. Elektrochirurgiegenerator gemäß dem vorhergehenden Anspruch, **dadurch gekennzeichnet, dass** die Spannungsquellen über Schalter mit den Primärwicklungen der Ausgangstransformatoren (40.1, 40.2, 40.3) verbunden werden können.

10. Elektrochirurgiegenerator gemäß wenigstens einem der vorhergehenden Ansprüche, bei dem die Schalter (S1, S2, S3, S4) Relais sind.

11. Elektrochirurgiegenerator gemäß wenigstens einem der vorhergehenden Ansprüche, der dazu konfiguriert ist, an seinen Ausgängen (20.1, 20.2) eine Ausgangswechselspannung mit einer Frequenz zwischen 0,2 MHz und 3 MHz bereitzustellen.

12. Elektrochirurgiegenerator gemäß wenigstens einem der vorhergehenden Ansprüche, der dazu konfiguriert ist, an seinen Ausgängen (20.1, 20.2) eine Ausgangswechselspannung zwischen 200 V und 5 kV bereitstellen.

13. Elektrochirurgiegenerator gemäß wenigstens einem der vorhergehenden Ansprüche, der dazu konfiguriert ist, an seinen Ausgängen (20.1, 20.2) eine Ausgangsleistung von bis zu 500 W bereitzustellen.

## Claims

1. Electrosurgical generator (10) for providing different high-frequency alternating voltages/high-frequency currents, comprising at least two outputs (20.1, 20.2) to which an electrosurgical instrument can be connected, at least one high-frequency voltage source (12), and at least two output transformers (40.1, 40.2, 40.3) each having at least one primary winding (42.1, 42.2, 42.3; 42.1', 42.2', 42.3') and one secondary winding (44.1, 44.2, 44.3; 44.1', 44.2', 44.3'), wherein the output transformers (40.1, 40.2, 40.3) are connected on the primary side to the high-frequency voltage source (12) and on the secondary side to the outputs (20.1, 20.2) for an electrosurgical instrument,
**characterized in that** the electrosurgical generator (10) comprises switching means (S1, S2, S2.1, S2.2, S3, S4; S4.1, S4.2, S5.1, S5.2), wherein
the primary windings (42.1, 42.2, 42.3; 42.1', 42.2', 42.3') of the output transformers (40.1, 40.2, 40.3) can each be optionally connected in parallel and/or in series or operated individually as well as the secondary windings (44.1, 44.2, 44.3; 44.1', 44.2', 44.3') of the output transformers (40.1, 40.2, 40.3) can each be optionally connected in parallel and/or in series or operated individually.

2. Electrosurgical generator according to the preceding claim, **characterized in that** at least one output transformer (40.1, 40.2, 40.3) has at least one tap on the primary and/or secondary side.

3. Electrosurgical generator according to one of the preceding claims, **characterized in that** the outputs of the secondary windings of the output transformers (40.1, 40.2, 40.3) can be connected via switching means to the outputs (20.1, 20.2) for an electrosurgical instrument.

4. Electrosurgical generator according to one of the preceding claims, **characterized in that** the activation of the switches (S1, S2, S2.1, S2.2, S3, S4, S4.1, S4.2, S5.1, S5.2) is performed via a control unit.

5. Electrosurgical generator according to one of claims 1 or 2 and 4, **characterized in that**, for setting four different transmission ratios between the high-frequency voltage source and the outputs (20.1, 20.2) for an electrosurgical instrument, three output transformers (40.1 , 40.2, 40.3) are provided, the windings of which can be connected both on the primary side and on the secondary side via two switches each.

6. Electrosurgical generator according to the preceding claim, **characterized in that** the transmission ratios of the two outer output transformers (40.1, 40.2, 40.3) are configured differently.

7. Electrosurgical generator according to the preceding claim, **characterized in that** the transmission ratio of the center output transformer (40.2) is of the order of the smaller transmission ratio of the outer output transformers (40.1, 40.3).

8. Electrosurgical generator according to at least one of the preceding claims 1 to 5, **characterized in that** the electrosurgical generator comprises at least two high-frequency voltage sources.

9. Electrosurgical generator according to the preceding claim, **characterized in that** the voltage sources can be connected via switches to the primary windings of the output transformers (40.1, 40.2, 40.3).

10. Electrosurgical generator according to at least one of the preceding claims, wherein the switches (S1, S2, S3, S4) are relays.

11. Electrosurgical generator according to at least one of the preceding claims that is configured to provide an AC output voltage at a frequency between 0.2 MHz and 3 MHz at its outputs (20.1, 20.2).

12. Electrosurgical generator according to at least one of the preceding claims that is configured to provide an AC output voltage between 200 V and 5 kV at its outputs (20.1, 20.2).

13. Electrosurgical generator according to at least one of the preceding claims that is configured to provide an output power of up to 500 W at its outputs (20.1, 20.2).

## Revendications

1. Générateur électro-chirurgical (10) permettant la fourniture de différentes tensions alternatives à haute fréquence / différents courants alternatifs à haute fréquence, avec au moins deux sorties (20.1, 20.2) auxquelles un instrument électro-chirurgical peut être raccordé, au moins une source de tension à haute fréquence (12) et au moins deux transformateurs de sortie (40.1, 40.2, 40.3) qui comportent chacun au moins un enroulement primaire (42.1, 42.2, 42.3 ; 42.1', 42.2', 42.3') et un enroulement secondaire (44.1, 44.2, 44.3 ; 44.1', 44.2', 44.3'), les transformateurs de sortie (40.1, 40.2, 40.3) étant reliés côté primaire à la source de tension à haute fréquence (12) et côté secondaire aux sorties (20.1, 20.2) pour un instrument électro-chirurgical, **caractérisé en ce que** le générateur électro-chirurgical (10) comporte des moyens de commutation (S1, S2, S2.1, S2.2, S3, S4 ; S4.1, S4.2, S5.1, S5.2) avec lesquels aussi bien les enroulements primaires (42.1, 42.2, 42.3 ; 42.1', 42.2', 42.3') des transformateurs de sortie (40.1, 40.2, 40. 3) peuvent être alimentés au choix en parallèle et/ou en série ou individuellement, ainsi que les enroulements secondaires (44.1, 44.2, 44.3 ; 44.1', 44.2', 44.3') des transformateurs de sortie (40.1, 40.2, 40.3) peuvent être alimentés au choix en parallèle et/ou en série ou individuellement.

2. Générateur électro-chirurgical selon la revendication précédente, **caractérisé en ce qu'**au moins un transformateur de sortie (40.1, 40.2, 40.3) comporte au moins une prise du côté primaire et/ou du côté secondaire.

3. Générateur électro-chirurgical selon l'une des revendications précédentes, **caractérisé en ce que** les sorties des enroulements secondaires des transformateurs de sortie (40.1, 40.2, 40.3) peuvent être reliées par des commutateurs aux sorties (20.1, 20.2) pour un instrument électro-chirurgical.

4. Générateur électro-chirurgical selon l'une des revendications précédentes, **caractérisé en ce que** la commande des moyens de commutation (S1, S2, S2.1, S2.2, S3, S4, S4.1, S4.2, S5.1, S5.2) a lieu par le biais d'une unité de commande.

5. Générateur électro-chirurgical selon l'une des revendications 1 ou 2 et 4, **caractérisé en ce que**, pour le réglage de quatre rapports de transformation différents entre la source de tension à haute fréquence et les sorties (20.1, 20.2) pour un instrument électro-chirurgical, il est prévu trois transformateurs de sortie (40.1, 40.2, 40.3) dont les enroulements peuvent être reliés aussi bien du côté primaire que du côté secondaire par respectivement deux commutateurs.

6. Générateur électro-chirurgical selon la revendication précédente, **caractérisé en ce que** les rapports de transformation des deux transformateurs de sortie extérieurs (40.1, 40.2, 40.3) sont conçus différemment.

7. Générateur électro-chirurgical selon la revendication précédente, **caractérisé en ce que** le rapport de transformation du transformateur de sortie central (40.2) est de l'ordre de grandeur du rapport de transformation plus petit des transformateurs de sortie extérieurs (40.1, 40.3).

8. Générateur électro-chirurgical selon au moins une des revendications précédentes 1 à 5, **caractérisé en ce que** le générateur électro-chirurgical comprend au moins deux sources de tension à haute fréquence.

9. Générateur électro-chirurgical selon la revendication précédente, **caractérisé en ce que** les sources de tension peuvent être reliées aux enroulements primaires des transformateurs de sortie (40.1, 40.2, 40.3) par l'intermédiaire de commutateurs.

10. Générateur électro-chirurgical selon au moins l'une des revendications précédentes, dont les commutateurs (S1, S2, S3, S4) sont des relais.

11. Générateur électro-chirurgical selon au moins l'une des revendications précédentes, configuré pour fournir sur ses sorties (20.1, 20.2) une tension alternative de sortie ayant une fréquence comprise entre 0,2 MHz et 3 MHz.

12. Générateur électro-chirurgical selon au moins l'une des revendications précédentes, configuré pour fournir à ses sorties (20.1, 20.2) une tension alternative de sortie comprise entre 200 V et 5 kV.

13. Générateur électro-chirurgical selon au moins l'une des revendications précédentes, configuré pour fournir à ses sorties (20.1, 20.2) une puissance de sortie pouvant atteindre 500 W.
